# EUROPEAN PATENT APPLICATION

(11) **EP 4 803 613 A1**
(43) Date of publication of application: **09.09.2026**
(21) Application number: 26160857.4
(22) Date of filing: 26.02.2026
(51) Int. Cl.: C12N 5/0783, C12N 9/12, C12N 15/86, C12N 15/867

(54) **METHODS OF TRANSDUCTION OF NATURAL KILLER (NK) CELLS WITH RETROVIRAL VECTORS**

(30) Priority: 03.03.2025 EP 25161202
(71) Applicant: Miltenyi Biotec B.V. & Co. KG, 51429 Bergisch Gladbach (DE)
(72) Inventor: CRAMER, Sophie, 51429 Bergisch Gladbach (DE); ZHANG, Congcong, 51429 Bergisch Gladbach (DE)
(74) Representative: Biervert, Christian

(57) **Abstract**

The present invention provides an in-vitro method for the generation of a composition comprising genetically modified NK cells, the method comprising the steps a) enrichment of NK cells from a sample comprising NK cells and other cells, b) genetic modification of said enriched NK cells by transduction with a retroviral vector, c) cultivation of said genetically modified NK cells, wherein said genetic modification step b) is performed in the presence of a tyrosine kinase (TK) inhibitor, wherein the step of genetic modification of said enriched NK cells comprise the addition of said TK inhibitor to said enriched NK cells not later than 24 hours after the contact of the retroviral vector with the enriched NK cells, and wherein said TK inhibitor is added up to 24 hours before said genetic modification step to said enriched NK cells.

## Description

### Field of the invention

The present invention relates generally to the field of generating genetically engineered NK cells, in particular NK cells transduced with retroviral vectors in the presence of a tyrosine kinase (TK) inhibitor.

### Background of the invention

Natural killer (NK) cells are large granular lymphocytes (LGL) and belong to the family of innate lymphoid cells (ILCs). NK cells possess natural cytotoxicity against transformed and stressed cells without prior sensitization. Their cytotoxicity is regulated by their germline encoded activating and inhibitory receptors to mediate non-MHC-restricted killing of tumor cells and virally infected cells. In the recent years, genetically modified NK cells have been attracting much attention for clinical use. NK cells genetically modified to express transgenes such as chimeric antigen receptors (CARs) or cytokines have shown antitumor activity in both pre-clinical and clinical studies (Berrien-Elliott et al. 2023, Blood, 141(8):856-868; Marin et al. 2024, Nat Med. 30(3):772-784; Li et al. 2023, Immunol Rev., 320(1):217-235). NK cell and genetically-modified NK cell manufacturing is a complex procedure with many steps, which may include cell isolation, activation, gene modification, expansion and harvest. One commonly used genetic modification method is viral transduction with lentiviral vectors or retroviral vectors. However, exposing NK cells to gene delivery vehicles such as viral vectors can negatively affect their viability and functionality. Therefore, maintaining and even enhancing viability and effector function of genetically modified NK cells during manufacturing is critical to their success in clinical applications..

Dasatinib (N-(2-Chloro-6-methylphenyl)-2-[[6-[4-(2-hydroxyethyl)-1-piperazinyl]-2-methyl-4-pyrimidinyl]amino]-5-thiazolecarboxamide) is an ATP-competitive protein tyrosine kinase inhibitor. The main targets of dasatinib are BCR/Abl (the "Philadelphia chromosome"), Src, c-Kit, ephrin receptors, and several other tyrosine kinases.

Lachota et al. (Lachota et al., 2018, Blood, 132 (Supplement 1): 3004) demonstrated dasatinib could inhibit the cytotoxicity and cytokine production of NK cells.

Chang et al. (Chang et al., 2019, Front Immunol., 17;9:3152) found that NK cells isolated from the dasatinib-treated CML patients in chronic phase (CP) had enhanced natural cytotoxicity. They attributed this effect to the downregulation of NKG2A on the surface of these NK cells.

Uchiyama et al. (Uchiyama et al., 2012, Hematol Oncol., 31(3):156-63) studied the effect of dasatinib on the in vitro proliferation and cytotoxicity of NK cells. They found that NK cells cultured with dasatinib exhibited higher proliferation and cytotoxicity compared with the control culture without dasatinib.

Huuhtanen et al. (Huuhtanen et al., 2022, Clin Invest., 1;132(17):e152585) performed immune monitoring for the patients with chronic-phase CML in a multicenter clinical trial combining dasatinib with IFN-a treatment. The addition of dasatinib increased both the frequency and absolute number of NK cells. However, the effector functions of these NK cells were reduced when treated with dasatinib in their study.

WO2023220632A1 described using dasatinib to produce a deactivated NK cell product prior to freezing to improve NK cell viability and cytotoxicity after thawing.

There is a need in the art for an improved or alternative method of transduction of NK cells with retroviral vectors.

### Brief description of the invention

The inventors found that NKG2D ligands were strongly upregulated on the surface of NK cells during the transduction process with retroviral vectors such as lentiviral vectors. Surprisingly, they found using a Tyrosine kinase (TK) inhibitor such as dasatinib during the NK cell manufacturing process, i.e. during the process of genetic modification of the NK cells by transduction with retroviral vectors, could significantly reduce the expression of NKG2D ligands on NK cell surface. Treatment of NK cells with dasatinib during this modification step results in higher NK cell viability. Surprisingly, chimeric antigen receptor (CAR) NK cells generated with the presence of dasatinib during the transduction process as disclosed herein exhibited enhanced in-vitro and/or in vivo NK cell viability and antitumor activity, compared to the non-dasatinib-treated NK cells expressing the same CAR construct.

Surprisingly, the TK inhibitors have no positive effect on the process of electroporation of NK cells, even though NKG2D ligands such as MICA/B were upregulated on NK cells due to the electroporation process.

Therefore, the present invention provides an in-vitro method for the generation of a composition comprising genetically modified NK cells, the method comprising the steps
a) enrichment of NK cells from a sample comprising NK cells and other cells,
b) genetic modification of said enriched NK cells by transduction with a retroviral vector such as a lentiviral vector,
c) cultivation of said genetically modified NK cells,
wherein said genetic modification step b) is performed in the presence of a tyrosine kinase (TK) inhibitor.

The use of a TK inhibitor such as dasatinib for genetically modifying NK cells by transduction with a retroviral vector such as a lentiviral vector is also disclosed.

### Brief description of the drawings

Figure 1: Schematic representation of the CAR constructs of CD123 CAR-bbz (A) and CD123 CAR-10z (B).
Figure 2: Flow cytometric analysis of NKG2DL expression on CAR NK cells transduced with lentiviral vectors. Untransduced (UTD) NK cells served as controls. Mean values ± SD are shown; n=3 individual donors. Data were analyzed by two-tailed paired Student's t-test. *, p < 0.05; ns: not significant (p > 0.05).
Figure 3: The effect of the presence of dasatinib during the transduction process on NKG2DL expression (A) and MICA/B expression (B) of CD123 CAR-bbz NK cells. Mean values ± SD are shown; n=3 individual donors. Data were analyzed by two-tailed paired Student's t-test. ***, p < 0.001; **, p < 0.01; *, p < 0.05; ns: not significant (p > 0.05).
Figure 4: The effect of the presence of dasatinib during the transduction process on NKG2DL expression (A) and MICA/B expression (B) of CD123 CAR-10z NK cells. Mean values ± SD are shown; n=3 individual donors. Data were analyzed by two-tailed paired Student's t-test. ***, p < 0.001; **, p < 0.01; *, p < 0.05; ns: not significant (p > 0.05).
Figure 5: The effect of the presence of dasatinib during the transduction process on the viability of CAR NK cells genetically modified with CD123 CAR-bbz (A) and CD123 CAR-10z (B) constructs. Mean values ± SD are shown; n=3 individual donors. Data were analyzed by 2-way ANOVA. **, p < 0.01.
Figure 6: The effect of the presence of dasatinib during the transduction process on the in vitro cytotoxicity of CAR NK cells. Each panel displays the data obtained from an individual donor.
Figure 7: Flow cytometric analysis of CAR expression on NK cells treated with or without dasatinib during the transduction process. Untransduced (UTD) NK cells served as a control.
Figure 8: The effect of the presence of dasatinib during the transduction process on the in vivo antitumor activity of CAR NK cells. Mean values ± SD are shown. Data were analyzed by 2-way ANOVA. ****, p < 0.0001; *, p < 0.05; ns: not significant (p > 0.05).
Figure 9: The effect of the presence of dasatinib during the transduction process on the proliferation of gene-modified NK cells. (A) The experiment was conducted using a range of 10 - 3000 nM of dasatinib. (B) The experiment was conducted using 0, 20, or 60 µM of dasatinib.
Figure 10: Dasatinib treatment during the electroporation process does not markedly reduce MICA/B expression of electroporated NK cells.
Figure 11: Dasatinib treatment during the electroporation process has negative effect on NK cell proliferation.
Figure 12: The effect of adding dasatinib at different time points during the NK transduction process on NK cell proliferation. Each panel displays the data obtained from an individual donor.

### Detailed description of the invention

In a first aspect the present invention provides an in-vitro method for the generation of a composition comprising genetically modified NK cells, the method comprising the steps
a) enrichment of NK cells from a sample comprising NK cells and other cells,
b) genetic modification of said enriched NK cells by transduction with a retroviral vector such as a lentiviral vector,
c) cultivation of said genetically modified NK cells,
wherein said genetic modification step b) is performed in the presence of a tyrosine kinase (TK) inhibitor (wherein a TK inhibitor is added during said step of genetic modification of said enriched NK cells).

Said TK inhibitor may be for e.g. dasatinib, bosutinib, nilotinib, imatinib or saracatinib. In a preferred embodiment said TK inhibitor is dasatinib.

Said comprising NK cells and other cells may be e.g. leukapheresis, buffy coat, whole blood, cord blood or peripheral blood mononuclear cells (PBMCs).

Said method, wherein said genetic modification of said enriched NK cells by transduction is the transduction (introduction) of a nucleic acid encoding a therapeutically effective protein such as a chimeric antigen receptor or a transgenic TCR.

Said method, wherein said step of genetic modification of said enriched NK cells comprise the addition of said NK inhibitor to said enriched NK cells not later than 4 hours, not later than 6 hours, not later than 12 hours, not later than 18 hours, not later than 24 hours, not later than 36 hours, not later than 48 hours or not later than 72 hours after the contact (addition) of the retroviral vector with (to) the enriched NK cells.

Said method, wherein said step of genetic modification of said enriched NK cells comprise the addition of said NK inhibitor to said enriched NK cells not later than 4 hours, not later than 6 hours, not later than 12 hours, not later than 18 hours, or not later than 24 hours after the contact (addition) of the retroviral vector with (to) the enriched NK cells, wherein said TK inhibitor is added up to 6 hours, up to 12 hours or 24 hours before said genetic modification step to said enriched NK cells.

Said method, wherein said step of genetic modification of said enriched NK cells comprise the addition of said NK inhibitor to said enriched NK cells not later than 24 hours after the contact (addition) of the retroviral vector with (to) the enriched NK cells, wherein said TK inhibitor is added up to 24 hours before said genetic modification step to said enriched NK cells.

In one embodiment of the invention the in-vitro method is a method for the generation of a composition comprising genetically modified NK cells, the method comprising the steps
a) enrichment of NK cells from a sample comprising NK cells and other cells,
b) genetic modification of said enriched NK cells by transduction with a retroviral vector such as a lentiviral vector,
c) cultivation of said genetically modified NK cells,
wherein said genetic modification step b) is performed in the presence of a tyrosine kinase (TK) inhibitor, and wherein said TK inhibitor is added up to 24 hours before said genetic modification step b) (but after said enrichment step a)) and not later than 24 hours after the contact (addition) of the retroviral vector with (to) the enriched NK cells.

Said method, wherein said TK inhibitor is also present (is also added) during the cultivation step c).

Said method, wherein said TK inhibitor is not present (is not added) during the cultivation step c).

In some embodiments, said TK inhibitor is removed by washing out the TK inhibitor by e.g one or more washing steps or by medium exchange. Such washings steps or media exchange may be performed e.g. after the transduction step and before the cultivation step and/or during the cultivation step.

Said method, wherein said cultivation of said genetically modified NK cells comprises the presence (the addition) of cytokine(s) such IL-2 and/or IL-15.

Said method, wherein the presence of said TK inhibitor with the NK cells is for about 1 hour to 72 hours, 1 hour to 48 hours, 1 hour to 36 hours, 1 hour to 24 hours, 1 hour to 12 hours, 1 hour to 6 hours, 2 hours to 5 hours or 3 hours to 4 hours.

Said method, wherein said TK inhibitor is added to the NK cells after the enrichment step a) but before the genetic modification step b), or said TK inhibitor is added to the NK cells concurrently with the genetic modification step b).

Said method, wherein said TK inhibitor is added to the NK cells during the genetic modification step b), wherein said genetic modification step comprises a spinoculation step, preferentially an about 2-hour spinoculation step, and wherein the addition of said TK inhibitor is performed
i) before said spinoculation step, wherein said TK inhibitor is added concurrently with the retroviral vector to the NK cells
ii) before said spinoculation step but after the addition of the retroviral vector to the NK cells, and/or
iii) after said spinoculation step but before said cultivation step c).

Said method, wherein said genetic modification step comprises 2, 3, 4, or 5 spinoculation steps within 72 hours of the contact of said retroviral vector with said NK cells.

Said method, wherein said spinoculation step is for about 10 minutes, for about 20 minutes, for about 30 minutes, for about 40 minutes, for about 50 minutes, for about 60 minutes, for about 70 minutes, for about 80 minutes, for about 90 minutes, for about 100 minutes, for about 120 minutes, or for about 180 minutes.

Said method, wherein said TK inhibitor is added to the NK cells during the genetic modification step b), wherein said genetic modification step comprises a spinoculation step, preferentially an about 2-hour spinoculation step, and wherein said addition of said TK inhibitor is performed
i) before said spinoculation step, wherein said TK inhibitor is added concurrently with the retroviral vector to the NK cells
ii) before said spinoculation step but after the addition of the retroviral vector to the NK cells, and/or
iii) after said spinoculation step but before said cultivation step c ), wherein said addition of said TK inhibitor is performed not later than 4 hours, not later than 6 hours, not later than 12 hours, not later than 18 hours, not later than 24 hours, not later than 36 hours, not later than 48 hours or not later than 72 hours after the contact (addition) of the retroviral vector with (to) the enriched NK cells.

Said method, wherein said TK inhibitor is added up to 24 hours before said genetic modification step to said enriched NK cells (but after said enrichment step), i.e. during a cultivation step between enrichment of NK cells and transduction of NK cells.

Said method, wherein said genetic modification step b) is performed on day 0, on day 1 , on day 2, on day 3, on day 4, on day 5, on day 6, on day 7, on day 8, on day 9 or on day 10 after said enrichment step a) (wherein said enrichment step a) is on day 0).

Said method, wherein said genetic modification step b) is performed on day 1 after said enrichment step a) (wherein said enrichment step a) is on day 0), and wherein said TK inhibitor is also present during the cultivation step c).

Said method, wherein said genetic modification step b) is performed on day 0, on day 1, on day 2, on day 3, on day 4, on day 5, on day 6, on day 7, on day 8, on day 9 or on day 10 after said enrichment step a) (wherein said enrichment step a) is on day 0), and wherein said TK inhibitor is also present during the cultivation step c), wherein said TK inhibitor is present at least on day 1, day 2 and/or day 3 of said cultivation step.

Said method, wherein said genetic modification step b) is performed on day 0, on day 1, on day 2, on day 3, on day 4, on day 5, on day 6, on day 7, on day 8, on day 9 or on day 10 after said enrichment step a) (wherein said enrichment step a) is on day 0), and
wherein said TK inhibitor is also present during the cultivation step c), wherein said TK inhibitor is present at least on day 1, day 2 and/or day 3 of said cultivation step,
wherein said TK inhibitor is also present up to 24 hours before said genetic modification step (but after said enrichment step a)).

Said method, wherein said genetic modification step b) is performed on day 0, on day 1, on day 2, on day 3, on day 4, on day 5, on day 6, on day 7, on day 8, on day 9 or on day 10 after said enrichment step a) (wherein said enrichment step a) is on day 0), and
wherein said TK inhibitor is also present during the cultivation step c), wherein said TK inhibitor is preferentially present at least on day 1 of said cultivation step,
wherein said TK inhibitor is also present up to 24 hours before said genetic modification step (but after said enrichment step a)).

Said method, wherein the concentration of said TK inhibitor (in the fluid/liquid/ cell medium that comprises the NK cells) in step b), and/or between step a) and b) and optionally additionally in step c) is between 10 nM and 20 µM, between 100 nM and 1µM, between 200 nM and 800 nM, or between 250 nM and 750 nM.

Said method, wherein said TK inhibitor is dasatinib.

Said method, wherein the concentration of said dasatinib (in the fluid/liquid/ cell medium that comprises the NK cells) in step b), and/or between step a) and b) and optionally additionally in step c) is between 10 nM and 20 µM, between 100 nM and 1000 nM, between 200 nM and 800 nM, or between 250 nM and 750 nM.

Said method, wherein said method comprises step d):
formulating the genetically-modified and cultured NK cells into a final cell composition, wherein said final cell composition formulation is (essentially) free of said TK inhibitor.

Said method, where said method additionally comprises a step of cryopreservation of the NK cells of said a final cell composition formulation.

Cryopreservation or cryoconservation is a process that preserves biological materials, such as organelles, cells, tissues, or any other biological samples, by subjecting the samples to very low temperatures, such as -80 °C (-112 °F) or -196 °C (-321 °F) using liquid nitrogen. Methods how to cryopreservate cells are well-known in the art.

In another aspect the present invention provides the use of a TK inhibitor such as dasatinib for genetically modifying NK cells by transduction with a retroviral vector such as a lentiviral vector.

Said use, wherein said genetically modified NK cells are cultured either in the presence or the absence of said TK inhibitor.

Said use, wherein the concentration of the TK inhibitor (in the fluid/liquid/cell medium that comprises the NK cells) for said transduction is between 10 nM and 20 µM, between 100 nM and 1000 nM, between 200 nM and 800 nM, or between 250 nM and 750 nM.

Said use, wherein the concentration of the TK inhibitor used during the transduction process is between 10 nM and 20 µM, between 100 nM and 1000 nM, between 200 nM and 800 nM, or between 250 nM and 750 nM.

Said use, wherein the concentration of dasatinib (in the fluid/liquid/cell medium that comprises the NK cells) for said transduction is between 10 nM and 20 µM, between 100 nM and 1000 nM, between 200 nM and 800 nM, or between 250 nM and 750 nM.

Said use, wherein the concentration of dasatinib used during the transduction process is between 10 nM and 20 µM, between 100 nM and 1000 nM, between 200 nM and 800 nM, or between 250 nM and 750 nM.

In a further aspect the present invention provides a composition comprising modified NK cells, wherein composition of modified NK cells are obtained by the in-vitro methods as disclosed herein.

Said composition, wherein said modified NK cells comprise a transgene encoding e.g. a CAR or a transgenic TCR. Said CAR may comprise an antigen binding domain specific for an antigen expressed on the surface of a target sell such as a cancer cell.

In another aspect the present invention provides a composition for use in treatment of a disease such as cancer, an autoimmune disease or an infectious disease, the composition comprising genetically modified NK cells, wherein said composition is obtained by the in-vitro methods as disclosed herein.

Said composition for use in treatment of a disease, wherein said modified NK cells comprise a transgene encoding e.g. a CAR or a transgenic TCR. Said CAR may comprise an antigen binding domain specific for an antigen on the surface of a target sell such as a cancer cell.

In another aspect the present invention provides a pharmaceutical composition comprising genetically modified NK cells, wherein said composition is obtained by the methods as disclosed herein, and optionally a pharmaceutically acceptable carrier.

Pharmaceutically acceptable carriers, diluents or excipients may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives.

Said pharmaceutical composition for use in treatment of a disease, wherein said modified NK cells comprise a transgene encoding e.g. a CAR or a transgenic TCR. Said CAR may comprise an antigen binding domain specific for an antigen on the surface of a target sell such as a cancer cell.

In one embodiment of the invention the composition comprises genetically modified NK cells, wherein said composition is obtained by the in vitro methods as disclosed herein, and wherein said NK cells have been modified to express a CAR as disclosed herein is for use in treatment of a disease associated with a target cell of a subject suffering from said disease such as a cancer, wherein said target cell may be a leukemia or lymphoma or solid cancer (said composition is referred to here as the "disclosed composition"). NK cells of a subject may be enriched as disclosed herein. The subject may e.g. suffer from said cancer or may be a healthy subject. These NK cells may be genetically modified in vitro to express the CAR as disclosed herein. These engineered NK cells of said disclosed composition may be activated and expanded in vitro. In a cellular therapy said disclosed composition may be infused to a recipient in need thereof. Said composition may be a pharmaceutical composition (said cell plus pharmaceutical acceptable carrier). The infused NK cells of said composition may be e.g. able to kill (or at least stop growth of) cancerous cells in the recipient. The recipient may be the same subject from which the cells was obtained (autologous cell therapy) or may be from another subject of the same species (allogeneic cell therapy).

Said composition comprising the NK cells engineered to express a CAR as disclosed herein may be administered either alone, or as a pharmaceutical composition in combination with diluents and/or with other components such as IL-2 or other cytokines or cell populations. Briefly, pharmaceutical compositions of said disclosed composition may be in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextran, mannitol; proteins; polypeptides or amino acids such as glycine; antioxidants; chelating agents such as EDTA or glutathione; adjuvants (e.g., aluminum hydroxide); and preservatives.

Preferentially, the disclosed compositions are formulated for intravenous administration. The administration of cell compositions to the subject may be carried out in any convenient manner known in the art.

Pharmaceutical compositions of the disclosed compositions may be administered in a manner appropriate to the disease to be treated. Appropriate dosages may be determined by clinical trials. But the quantity and frequency of administration will also be determined and influenced by such factors as the condition of the patient, and the type and severity of the patient's disease.

A pharmaceutical composition comprising the disclosed composition comprising the NK cells as disclosed herein may be administered at a dosage of 10⁴ to 10⁹ cells/kg body weight, preferably 10⁵ to 10⁷ cells/kg body weight. The disclosed compositions may also be administered several times at these dosages. The disclosed compositions of cells may be injected e.g. directly into a tumor, lymph node, or site of infection.

The disclosed composition may be used in combination with e.g. chemotherapy, radiation, immunosuppressive agents, antibodies or antibody therapies.

### Generation of NK cells

Processes of generation of a composition of NK cells are well known in the art. Exemplarily in the following methods of generation of immune cells expressing a CAR are disclosed.

Generally, other processes of enrichment of NK cells than the method disclosed herein are known in the art and such enrichments of NK cells may be performed by e.g. enrichment of CD56+ cells from a sample obtained from a subject such as a blood sample or PBMC, followed by depletion of CD3+ cells from said sample. Alternatively said enrichment of NK cells may be the CD3 depletion of cells from said sample. Alternatively said enrichment of NK cells may be the CD3 and CD19 and/or CD14 depletion of cells from said sample. Alternatively, said enrichment may be the depletion of CD3+ cells followed by the enrichment of CD56+ cells from said sample.

NK cells can be further cultivated and expanded by methods known in the art with cytokines such as IL-2, IL-15, IL-21, IL-12, IL-18, IL-1 family, and/or with feeder cells or artificial antigen presenting cells (APCs) such as K562-based feeder cells, EBV transformed lymphoblastoid cell lines, PMBCs, and/or cell-free derivatives such as cell particles or exosomes, and/or with stimulating reagents such as NK cell activation/expansion kit from Miltenyi Biotec, ImmunoCult^{™} NK Cell Expansion Kit from StemCell Technologies, CellXVivo Human NK Cell Expansion Kit from R&D Systems. NK cells can be cultivated and expanded by methods known in the art with culturing flasks, culturing bags, G-Rex, bioreactors, wave cell culture system, etc.

### Modification of NK cells

### Nucleotides, Expression, Vectors

The nucleic acids encoding a transgene such as a CAR as used herein may e.g. comprise a nucleotide sequence encoding any of the leader sequences, antigen binding domains, transmembrane domains, and/or intracellular T cell signaling domains described herein for CARs.

In some embodiments, the nucleotide sequence may be codon-modified. Without being bound to a particular theory, it is believed that codon optimization of the nucleotide sequence increases the translation efficiency of the mRNA transcripts. Codon optimization of the nucleotide sequence may involve substituting a native codon for another codon that encodes the same amino acid, but can be translated by tRNA that is more readily available within a cell, thus increasing translation efficiency. Optimization of the nucleotide sequence may also reduce secondary mRNA structures that would interfere with translation, thus increasing translation efficiency.

"Nucleic acid" as used herein includes "polynucleotide", "oligonucleotide", "nucleic acid molecule" and "nucleic acid sequence" and generally means a polymer of DNA or RNA, which can be single-stranded or double-stranded, synthesized or obtained (e.g., isolated and/or purified) from natural sources, which can contain natural, non-natural or altered nucleotides, and which can contain a natural, non-natural or altered internucleotide linkage, such as a phosphoroamidate linkage or a phosphorothioate linkage, instead of the phosphodiester found between the nucleotides of an unmodified oligonucleotide.

In an embodiment, the nucleic acids can be incorporated into a recombinant expression vector. In this regard, an embodiment provides recombinant expression vectors comprising any of the nucleic acids.

For purposes herein, the term "recombinant expression vector" means a genetically-modified oligonucleotide or polynucleotide construct that permits the expression of an mRNA, protein, polypeptide, or peptide by a host cell, when the construct comprises a nucleotide sequence encoding the mRNA, protein, polypeptide, or peptide, and the vector is contacted with the cell under conditions sufficient to have the mRNA, protein, polypeptide, or peptide expressed within the cell. The vectors are not naturally-occurring as a whole.

In an embodiment, the recombinant expression vector can be any suitable recombinant expression vector, and can be used to transform or transfect any suitable host cell. Suitable vectors include those designed for propagation and expansion or for expression or both, such as plasmids and viruses.

The recombinant expression vector may be a viral vector, e.g., a retroviral vector or a lentiviral vector. A lentiviral vector is a vector derived from at least a portion of a lentivirus genome, including especially a self-inactivating lentiviral vector. Other examples of lentivirus vectors that may be used in the clinic, include, for example, and not by way of limitation, the LENTIVECTOR.RTM. gene delivery technology from Oxford BioMedica plc, the LENTIMAX.TM. vector system from Lentigen and the like. Nonclinical types of lentiviral vectors are also available and would be known to one skilled in the art.

A number of transfection techniques are generally known in the art. Transfection methods include e.g. calcium phosphate co-precipitation, direct micro injection into cultured cells, electroporation, liposome mediated gene transfer, and lipid mediated transduction. If DNA or RNA is introduced into cells by using viral vector carriers, then the technique is called transduction.

Constructs of expression vectors, which are circular or linear, can be prepared to contain a replication system functional in a prokaryotic or eukaryotic host cell.

The recombinant expression vector may comprise regulatory sequences, such as transcription and translation initiation and termination codons, which are specific to the type of host cell (e.g., bacterium, fungus, plant, or animal) into which the vector is to be introduced, as appropriate, and taking into consideration whether the vector is DNA- or RNA-based. The recombinant expression vector may comprise restriction sites to facilitate cloning.

The recombinant expression vector can include one or more marker genes, which allow for selection of transformed or transfected host cells. Marker genes include biocide resistance, e.g., resistance to antibiotics, heavy metals, etc., complementation in an auxotrophic host to provide prototrophy, and the like. Suitable marker genes for the inventive expression vectors include, for instance, neomycin/G418 resistance genes, hygromycin resistance genes, histidinol resistance genes, tetracycline resistance genes, and ampicillin resistance genes.

The recombinant expression vector can comprise a native or nonnative promoter operably linked to the nucleotide sequence encoding the CAR (including functional portions and functional variants thereof), or to the nucleotide sequence which is complementary to or which hybridizes to the nucleotide sequence encoding the CAR. The selection of promoters, e.g., strong, weak, inducible, tissue-specific and developmental-specific, is within the ordinary skill of the artisan. Similarly, the combining of a nucleotide sequence with a promoter is also within the skill of the artisan. The promoter can be a nonviral promoter or a viral promoter, e.g., a cytomegalovirus (CMV) promoter, an SV40 promoter, an RSV promoter, human elongation factor-1 alpha (EF-1 alpha) promoter or a promoter found in the long-terminal repeat of the murine stem cell virus.

The recombinant expression vectors can be designed for either transient expression, for stable expression, or for both. Also, the recombinant expression vectors can be made for constitutive expression or for inducible expression.

Further, the recombinant expression vectors can be made to include a suicide gene. As used herein, the term "suicide gene" refers to a gene that causes the cell expressing the suicide gene to die. The suicide gene can be a gene that confers sensitivity to an agent, e.g., a drug, upon the cell in which the gene is expressed, and causes the cell to die when the cell is contacted with or exposed to the agent. Suicide genes are known in the art and include, for example, the Herpes Simplex Virus (HSV) thymidine kinase (TK) gene, cytosine deaminase, purine nucleoside phosphorylase, and nitroreductase.

### Processes for modification of NK cells

The composition of NK cells obtained by the method as disclosed herein may be genetically modified to express one or more transgenes.

Processes of genetically modifying NK cells are well known in the art. Exemplarily in the following methods of generation of NK cells expressing a transgene such as a CAR are disclosed.

A process for the generation of genetically modified NK cells with a pseudotyped retroviral vector is disclosed e.g. in WO2019121945A1 and may comprise the e.g. steps:
a) Activation of NK cells, and
b) Addition of a pseudotyped retroviral vector to said activated NK cells,

wherein said pseudotyped retroviral vector comprises a modified baboon endogenous retrovirus (BaEV) envelope glycoprotein that is able of binding to and fusing with a hematopoietic cell membrane, thereby transferring biological material into said activated NK cells,
wherein said modified baboon endogenous retrovirus (BaEV) envelope glycoprotein that is able of binding to and fusing with a hematopoietic cell membrane is:
   a chimeric envelope glycoprotein which comprises or consists in a fusion of the transmembrane and extracellular domain of a baboon endogenous retrovirus (BaEV) envelope glycoprotein and the cytoplasmic tail domain of a murine leukemia virus (MLV) envelope glycoprotein; or
   a modified BaEV envelope glycoprotein wherein the cytoplasmic tail domain is devoid of the fusion inhibitory R peptide,
   and wherein said pseudotyped retroviral vector comprises a nucleic acid encoding a transgene product such as a CAR.

Said activation of said NK cells may be achieved by the addition of at least one cytokine or feeder cells or membrane particles of feeder cells or a with an NK cell activation reagent to said NK cells.

Said at least one cytokine may be IL-2 and/or IL-15.

Said activation of NK cells may be achieved by the addition of a combination of cytokines comprising at least one cytokine that activates NK cells and a IL-1 family cytokine.

Said combination of cytokines may be IL2 and/or IL-15 and a IL-1 family cytokine.

Said IL-1 family cytokine may be IL-18, IL-33 or IL-1beta.

### General methods of treatment using the compositions as disclosed herein

It is contemplated that the composition of enriched NK cells and subsequently genetically modified as disclosed herein can be used in methods of treating or preventing a disease in a mammal. In this regard, an embodiment provides a method of treating cancer administering to the mammal (the subject) the composition of enriched NK cells and subsequently genetically modified as disclosed herein, wherein the modified NK cells express a CAR or TCR specific for an TAA of said cancer. The compositions may comprise modified NK cells in an amount effective to treat cancer in the mammal.

An embodiment, especially for the treatment of cancer, further comprises lymphodepleting the mammal prior to administering the compositions disclosed herein. Examples of lymphodepletion include, but may not be limited to, nonmyeloablative lymphodepleting chemotherapy, myeloablative lymphodepleting chemotherapy, total body irradiation, etc.

For purposes of the methods, wherein NK cells are administered, the cells can be cells that are allogeneic or autologous to the mammal. Preferably, the cells are allogeneic to the mammal. As used herein, allogeneic means any material derived from a different animal of the same species as the individual to whom the material is introduced. Two or more individuals are said to be allogeneic to one another when the genes at one or more loci are not identical. In some aspects, allogeneic material from individuals of the same species may be sufficiently unlike genetically to interact antigenically. As used herein, "autologous" means any material derived from the same individual to whom it is later to be re-introduced into the individual.

The mammal referred to herein can be any mammal. As used herein, the term "mammal" refers to any mammal, including, but not limited to, mammals of the order Rodentia, such as mice and hamsters, and mammals of the order Logomorpha, such as rabbits. The mammals may be from the order Carnivora, including Felines (cats) and Canines (dogs). The mammals may be from the order Artiodactyla, including Bovines (cows) and Swines (pigs) or of the order Perssodactyla, including Equines (horses). The mammals may be of the order Primates, Ceboids, or Simoids (monkeys) or of the order Anthropoids (humans and apes). Preferably, the mammal is a human.

The terms "treat," and "prevent" as well as words stemming therefrom, as used herein, do not necessarily imply 100% or complete treatment or prevention. Rather, there are varying degrees of treatment or prevention of which one of ordinary skill in the art recognizes as having a potential benefit or therapeutic effect. In this respect, the methods can provide any amount or any level of treatment of cancer in a mammal.

Any method of administration can be used for the disclosed therapeutic agents, including local and systemic administration. For example topical, oral, intravascular such as intravenous, intramuscular, intraperitoneal, intranasal, intradermal, intrathecal and subcutaneous administration can be used. The particular mode of administration and the dosage regimen will be selected by the attending clinician, taking into account the particulars of the case (for example the subject, the disease, the disease state involved, and whether the treatment is prophylactic). In cases in which more than one agent or composition is being administered, one or more routes of administration may be used; for example, a chemotherapeutic agent may be administered orally and a composition of NK cells expressing a CARs as disclosed herein may be administered intravenously.

Methods of administration include injection for which the compositions as disclosed herein are provided in a nontoxic pharmaceutically acceptable carrier such as water, saline, Ringer's solution, dextrose solution, 5% human serum albumin, fixed oils, ethyl oleate, or liposomes. In some embodiments, local administration of the disclosed compounds or compositions (e.g. the NK cells expressing a CAR as disclosed herein) can be used, for instance by applying the compounds or compositions to a region of tissue from which a tumor has been removed, or a region suspected of being prone to tumor development. In some embodiments, sustained intra-tumoral (or near-tumoral) release of the pharmaceutical preparation that includes a therapeutically effective amount of the compounds or compositions may be beneficial. In other examples, the conjugate is applied as an eye drop topically to the cornea, or intravitreally into the eye.

The disclosed therapeutic agents can be formulated in unit dosage form suitable for individual administration of precise dosages. In addition, the disclosed therapeutic agents may be administered in a single dose or in a multiple dose schedule. A multiple dose schedule is one in which a primary course of treatment may be with more than one separate dose, for instance 1-10 doses, followed by other doses given at subsequent time intervals as needed to maintain or reinforce the action of the compositions.

Treatment can involve daily or multi-daily doses of compound(s) over a period of a few days to months, or even years. Thus, the dosage regime will also, at least in part, be determined based on the particular needs of the subject to be treated and will be dependent upon the judgment of the administering practitioner.

All definitions, characteristics and embodiments defined herein with regard to the first aspect of the invention as disclosed herein also apply mutatis mutandis in the context of the other aspects of the invention as disclosed herein.

### Definitions

Unless defined otherwise, technical and scientific terms used herein have the same meaning as commonly understood by one of ordinary skill in the art to which this invention belongs.

As used herein the term "comprising" or "comprises" is used in reference to compositions, methods, and respective component(s) thereof, that are essential to the method or composition, yet open to the inclusion of unspecified elements, whether essential or not.

By "consisting of' is meant including, and limited to, whatever follows the phrase "consisting of." Thus, the phrase "consisting of' indicates that the listed elements are required or mandatory, and that no other elements may be present.

As used herein, the term "about" will be understood by persons of ordinary skill in the art and will vary to some extent on the context in which it is used. As used herein, "about" when referring to a measurable value such as an amount, a temporal duration, and the like, is meant to encompass variations of ±20% or ±10%, more preferably ±5%, even more preferably ±1% from the specified value

The TK inhibitor as disclosed herein may be Lorlatinib, Brigatinib, Ceritinib, Alectinib, Crizotinib, Bosutinib, Ponatinib, Nilotinib, Dasatinib, Saracatinib, Imatinib, Zanubrutinib, Acalabrutinib, Ibrutinib, Capmatinib, Pexidartinib, Dacomitinib, Osimertinib, Erlotinib, Gefitinib, Lapatinib, Afatinib, Pemigatinib, Erdafitinib, Nintedanib, Gilteritinib, Midostaurin, Tucatinib, Neratinib, Baricitinib, Ruxolitinib, Fedratinib, Tofacitinib, Ripretinib, Selumetinib, Binimetinib, Cobimetinib, Trametinib, Upadacitinib, Avapritinib, Selpercatinib, Cabozantinib, Fostamatinib, Larotrectinib, Entrectinib, Axitinib, Regorafenib, Pazopanib, Sorafenib, Lenvatinib, Vandetanib, and/or Sunitinib.

In certain embodiments, a TK inhibitor may be an anaplastic lymphoma kinase (ALK) inhibitor. In certain embodiments, an ALK inhibitor is Lorlatinib, Brigatinib, Ceritinib, Alectinib, and/or Crizotinib.

In certain embodiments, a TK inhibitor may be a Break Point Cluster Tyrosine-protein kinase ABL1 fusion (BCR-Abl) inhibitor. In certain embodiments, a BCR-Abl inhibitor is Bosutinib, Ponatinib, Nilotinib, Dasatinib, Saracatinib, and/or Imatinib. In certain embodiments, the TK inhibitor is Dasatinib and/or Nilotinib. In certain embodiments the TK inhibitor is Dasatinib.

In certain embodiments, a TK inhibitor may be a Bruton tyrosine kinase (BTK) inhibitor. In certain embodiments, a BTK inhibitor is Zanubrutinib, Acalabrutinib, and/or Ibrutinib.

In certain embodiments, a TK inhibitor may be a c-MET (a member of the MNNG HOS transforming gene family) inhibitor. In some embodiments, a c-MET inhibitor is Capmatinib. In certain embodiments, a TK inhibitor may be a colony stimulating factor 1 receptor (CSFR1) inhibitor. In some embodiments, a CSFR1 inhibitor is Pexidartinib.

In certain embodiments, a TK inhibitor may be an epidermal growth factor receptor (EGFR) inhibitor. In certain embodiments, an EGFR inhibitor is Dacomitinib, Osimertinib, Erlotinib, Gefitinib, Lapatinib, and/or Afatinib.

In certain embodiments, a TK inhibitor may be a fibroblast growth factor receptor (FGFR) inhibitor. In certain embodiments, a FGFR inhibitor is Pemigatinib, Erdafitinib, and/or Nintedanib.

In certain embodiments, a TK inhibitor may be a Vascular endothelial growth factor receptor (VEGFR) inhibitor. In certain embodiments, a VEGFR inhibitor is Gilteritinib, Axitinib, Regorafenib, Pazopanib, Sorafenib, Lenvatinib, Cabozantinib, and/or Vandetanib.

In certain embodiments, a TK inhibitor may be a fms-like tyrosine kinase 3 (FLT3) inhibitor. In certain embodiments, a FLT3 inhibitor is Midostaurin.

In certain embodiments, a TK inhibitor may be a Receptor tyrosine-protein kinase erbB- 2 (aka HER2) inhibitor. In some embodiments, a HER2 inhibitor is Tucatinib, Lapatinib, Afatinib, and/or Neratinib.

In certain embodiments, a TK inhibitor may be a Janus Kinase 1, 2, and/or 3 (JAK) inhibitor. In some embodiments, a JAK inhibitor is Baricitinib, Ruxolitinib, Fedratinib, and/or Tofacitinib. In certain embodiments, a TK inhibitor may be a platelet-derived growth factor receptor (PDGFR) inhibitor. In certain embodiments, a PDGFR inhibitor is Ripretinib, Upadacitinib, and/or Avapritinib.

In certain embodiments, a TK inhibitor may be a mitogen-activated protein kinase kinase 1/2 (MEK1/2) inhibitor. In some embodiments, a MEK1/2 inhibitor is Selumetinib, Binimetinib, Cobimetinib, and/or Trametinib.

In certain embodiments, a TK inhibitor may be proto-oncogene tyrosine-protein kinase receptor (RET) inhibitor. In certain embodiments, a RET inhibitor is Alectinib, Lenvatinib, Selpercatinib and/or Cabozantinib.

In certain embodiments, a TK inhibitor may be a tyrosine-protein kinase (SYK) inhibitor. In some embodiments, a SYK inhibitor is Fostamatinib

In certain embodiments, a TK inhibitor may be a Trk system potassium uptake protein A, B, and/or C (TRKA/B/C) inhibitor. In some embodiments, a TRKA/B/C inhibitor is Larotrectinib, and/or Entrectinib.

In certain embodiments, a TK inhibitor may be a proto-oncogene tyrosine-protein kinase ROS (ROS1) inhibitor. In some embodiments, a ROS1 inhibitor is Crizotinib, and/or Entrectinib.

In certain embodiments, a TK inhibitor may be a mast/stem cell growth factor receptor Kit (KIT) inhibitor. In certain embodiments, a KIT inhibitor is Ripretinib, and/or Imatinib.

Dasatinib, sold under the brand name Sprycel among others, is a targeted therapy medication used to treat certain cases of chronic myelogenous leukemia (CML) and acute lymphoblastic leukemia (ALL). Dasatinib is an ATP-competitive protein tyrosine kinase inhibitor. The main targets of dasatinib are BCR/Abl (the "Philadelphia chromosome"), Src, c-Kit, ephrin receptors, and several other tyrosine kinases.

The term "antibody" as used herein is used in the broadest sense to cover the various forms of antibody structures including but not being limited to monoclonal and polyclonal antibodies (including full length antibodies), multispecific antibodies (e.g. bispecific antibodies), antibody fragments, i.e. antigen binding fragments of an antibody, immunoadhesins and antibody-immunoadhesin chimeras, that specifically recognize (i.e. bind) an antigen. "Antigen binding fragments" comprise a portion of a full-length antibody, preferably the variable domain thereof, or at least the antigen binding site thereof ("an antigen binding fragment of an antibody"). Examples of antigen binding fragments include Fab (fragment antigen binding), scFv (single chain fragment variable), single domain antibodies (nanobodies), diabodies, dsFv, Fab', F(ab')2, diabodies, single-chain antibody molecules, and multispecific antibodies formed from antibody fragments. The antibody or antibody fragment may be human, fully human, humanized, human engineered, non-human, and/or chimeric. The non-human antibody or antibody fragment may be humanized to reduce immunogenicity to humans, while retaining the specificity and affinity of the parental non-human antibody. Chimeric antibodies may refer to antibodies created through the joining of two or more antibody genes which originally encoded for separate antibodies.

The terms "having specificity for", "specifically binds" or "specific for" with respect to an antigen-binding domain of an antibody, of a fragment thereof or of a CAR refer to an antigen-binding domain which recognizes and binds to a specific antigen, but does not substantially recognize or bind other molecules in a sample. An antigen-binding domain that binds specifically to an antigen from one species may bind also to that antigen from another species. This cross-species reactivity is not contrary to the definition of that antigen-binding domain is specific. An antigen-binding domain that specifically binds to an antigen may bind also to different allelic forms of the antigen (allelic variants, splice variants, isoforms etc.). This cross reactivity is not contrary to the definition of that antigen-binding domain is specific.

As used herein, the term "antigen" is intended to include substances that bind to or evoke the production of one or more antibodies and may comprise, but is not limited to, proteins, peptides, polypeptides, oligopeptides, lipids, carbohydrates such as dextran, haptens and combinations thereof, for example a glycosylated protein or a glycolipid. The term "antigen" as used herein refers to a molecular entity that may be expressed e.g. on the surface of a target cell and that can be recognized by means of the adaptive immune system including but not restricted to antibodies or TCRs, or engineered molecules including but not restricted to endogenous or transgenic TCRs, CARs, scFvs or multimers thereof, Fab-fragments or multimers thereof, antibodies or multimers thereof, single chain antibodies or multimers thereof, or any other molecule that can execute binding to a structure with high affinity.

The terms "immune cell" or "immune effector cell" may be used interchangeably and refer to a cell that may be part of the immune system and executes a particular effector function such as alpha-beta T cells, NK cells, NKT cells, B cells, innate lymphoid cells (ILC), cytokine induced killer (CIK) cells, lymphokine activated killer (LAK) cells, gamma-delta T cells, regulatory T cells (Treg), monocytes or macrophages. Preferentially these immune cells are human immune cells. Preferred immune cells are cells with cytotoxic effector function such as alpha-beta T cells, NK cells, NKT cells, ILC, CIK cells, LAK cells or gamma-delta T cells. Most preferred immune effector cells are T cells and NK cells. Tumor infiltrating lymphocytes (TILs) are T cells that have moved from the blood of a subject into a tumor. These TILs may be removed from a patient's tumor by methods well known in the art, e.g. enzymatic and mechanic tumor disruption followed by density centrifugation and/or cell marker specific enrichment. TILs are genetically engineered as disclosed herein, and then given back to the patient. "Effector function" means a specialized function of a cell, e.g. in a T cell an effector function may be cytolytic activity or helper activity including the secretion of cytokines.

Immunotherapy is a medical term defined as the "treatment of disease by inducing, enhancing, or suppressing an immune response". Immunotherapies designed to elicit or amplify an immune response are classified as activation immunotherapies, while immunotherapies that reduce or suppress are classified as suppression immunotherapies. Cancer immunotherapy as an activating immunotherapy attempts to stimulate the immune system to reject and destroy tumors. Adoptive cell transfer uses cell-based, preferentially T cell-based or NK cell-based cytotoxic responses to attack cancer cells. For example, T cells or NK cells that have a natural or genetically engineered reactivity to a patient's cancer are generated in-vitro and then transferred into the cancer patient. Then the immunotherapy is referred to as "CAR cell immunotherapy" or in case of use of T cells only as "CAR T cell therapy" or "CAR T cell immunotherapy" or in case of use of NK cells only as "CAR NK cell therapy" or "CAR NK cell immunotherapy"..

The term "treatment" as used herein means to reduce the frequency or severity of at least one sign or symptom of a disease.

The term "autologous" as used herein refers to any material derived from the same subject to who it is later re-introduced.

The term "allogeneic" as used herein refers to any material derived from a different subject of the same species as the subject to who the material is re-introduced.

The terms "therapeutically effective amount" or "therapeutically effective population" mean an amount of a cell population which provides a therapeutic benefit in a subject.

As used herein, the term "subject" refers to an animal. Preferentially, the subject is a mammal such as mouse, rat, cow, pig, goat, chicken dog, monkey or human. More preferentially, the subject is a human. The subject may be a subject suffering from a disease such as cancer (a patient) or from an autoimmune disease or from an allergic disease or from an infectious disease or from graft rejection.

The term "expression" as used herein is defined as the transcription of a particular nucleotide sequence into RNA and optionally subsequent translation of said RNA into a polypeptide sequence or a protein.

A "vector" comprises a (isolated) nucleic acid molecule which can be used to deliver the (isolated) nucleic acid molecule to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid. The term should also be construed to include non-plasmid and non-viral compounds which facilitate transfer of nucleic acid into cells, such as, for example, polylysine compounds, liposomes, and the like. Examples of viral vectors include, but are not limited to, adenoviral vectors, adeno-associated virus vectors, retroviral vectors, and the like. Said vector may be preferentially a retroviral vector such as a lentiviral vector.

The terms "engineered cell" and "genetically modified cell" as used herein can be used interchangeably. The terms mean containing and/or expressing a foreign gene or nucleic acid sequence which in turn modifies the genotype or phenotype of the cell or its progeny. Especially, the terms refer to the fact that cells, preferentially T cells or NK cells can be manipulated by recombinant methods well known in the art to express stably or transiently peptides or proteins which are not expressed in these cells in the natural state.

The terms "nucleic acid", "nucleic acid sequence" or "polynucleotide" as used interchangeably herein refer to polymers of nucleotides. Polynucleotides, which can be hydrolyzed into monomeric "nucleotides." The monomeric nucleotides can be hydrolyzed into nucleosides. As used herein, the term "polynucleotides" encompasses, but is not limited to, all nucleic acid sequences which are obtained by any means available in the art, including, without limitation, recombinant means, i.e., the cloning of nucleic acid sequences from a recombinant library or a cell genome, using ordinary cloning technology and PCR, and the like, and by synthetic means. The T cell receptor (TCR) is a protein complex found on the surface of T cells, or T lymphocytes, that is responsible for recognizing fragments of antigen as peptides bound to major histocompatibility complex (MHC) molecules. However, TCRs can also be non-MHC-restricted, such as most γδ TCRs and TCRs employed by invariant natural killer T cells.

The TCR is composed of two different protein chains (that is, it is a heterodimer). In humans, in 95% of T cells the TCR consists of an alpha (α) chain and a beta (β) chain (encoded by TRA and TRB, respectively), whereas in 5% of T cells the TCR consists of gamma and delta (γ/δ) chains (encoded by TRG and TRD, respectively). This ratio changes during ontogeny and in diseased states (such as leukemia). Each locus can produce a variety of polypeptides with constant and variable regions.

For enrichment, isolation or selection in principle any sorting technology can be used. This includes for example affinity chromatography or any other antibody-dependent separation technique known in the art. Any ligand-dependent separation technique known in the art may be used in conjunction with both positive and negative separation techniques that rely on the physical properties of the cells. An especially potent sorting technology is magnetic cell sorting. Methods to separate cells magnetically are commercially available e.g. from Invitrogen, Stem cell Technologies, in Cellpro, Seattle or Advanced Magnetics, Boston. For example, monoclonal antibodies can be directly coupled to magnetic polystyrene particles like Dynal M 450 or similar magnetic particles and used e.g. for cell separation. The Dynabeads technology is not column based, instead these magnetic beads with attached cells enjoy liquid phase kinetics in a sample tube, and the cells are isolated by placing the tube on a magnetic rack. However, in a preferred embodiment for enriching NK cells from a sample comprising NK cells according the present invention monoclonal antibodies or antigen binding fragments thereof are used in conjunction with colloidal superparamagnetic microparticles having an organic coating by e.g. polysaccharides (Magnetic-activated cell sorting (MACS) technology (Miltenyi Biotec B.V. & Co. KG, Germany)). These particles (nanobeads or MicroBeads) can be either directly conjugated to monoclonal antibodies or used in combination with anti-immunoglobulin, avidin or anti-hapten-specific MicroBeads.

The MACS technology allows cells to be separated by incubating them with magnetic nanoparticles coated with antibodies directed against a particular surface antigen. This causes the cells expressing this antigen to attach to the magnetic nanoparticles. Afterwards the cell solution is transferred on a column placed in a strong magnetic field. In this step, the cells attach to the nanoparticles (expressing the antigen) and stay on the column, while other cells (not expressing the antigen) flow through. With this method, the cells can be separated positively or negatively with respect to the particular antigen(s)/marker(s).

In case of a positive selection the cells expressing the antigen(s) of interest, which attached to the magnetic column, are washed out to a separate vessel, after removing the column from the magnetic field.

In case of a negative selection the antibody used is directed against surface antigen(s) which are known to be present on cells that are not of interest. After application of the cells/magnetic nanoparticles solution onto the column the cells expressing these antigens bind to the column and the fraction that goes through is collected, as it contains the cells of interest. As these cells are non-labelled by an antibody coupled to nanoparticles, they are "untouched".

The procedure can be performed using direct magnetic labelling or indirect magnetic labelling. For direct labelling the specific antibody is directly coupled to the magnetic particle. Indirect labelling is a convenient alternative when direct magnetic labelling is not possible or not desired. A primary antibody, a specific monoclonal or polyclonal antibody, a combination of primary antibodies, directed against any cell surface marker can be used for this labelling strategy. The primary antibody can either be unconjugated, biotinylated, or fluorophore-conjugated. The magnetic labelling is then achieved with anti-immunoglobulin MicroBeads, anti-biotin MicroBeads, or anti-fluorophore MicroBeads.

The term "cells that are CD3+" or "cells are CD3 positive" or "CD3 positive cells" may be used interchangeable and means that these cells are cells that express the antigen (marker) CD3 on their cell surface. This allows the select/sort cells that express this marker from cells that do not express this marker. It is understood that in the same way this applies to other markers such as CD14, CD19 or CD56.

The term "natural killer cells (NK cells)" are defined as large granular lymphocytes (LGL) and constitute the third kind of cells differentiated from the common lymphoid progenitor-generating B and T lymphocytes. NK cells are known to differentiate and mature in the bone marrow, lymph nodes, spleen, tonsils, and thymus, where they then enter the circulation. NK cells differ from natural killer T cells (NKTs) phenotypically, by origin and by respective effector functions; often, NKT cell activity promotes NK cell activity by secreting IFNγ. In contrast to NKT cells, NK cells do not express T-cell antigen receptors (TCR) or pan T marker CD3 or surface immunoglobulins (Ig) B cell receptors, but they usually express the surface markers CD16 (FcγRIII) and CD56 in humans, NK1.1 or NK1.2 in C57BL/6 mice. A subset of human NK cells also express CD8. Continuously growing NK cell lines can be established from cancer patients and common NK cell lines are for instance NK-92, NKL and YTS.

The term "isolated" means altered or removed from the natural state. For example an isolated population of cells means an enrichment of such cells and separation from other cells which are normally associated in their naturally occurring state with said isolated cells. An isolated population of cells means a population of substantially purified cells which are a homogenous population of cells.

As used herein the term "culturing" or "cultivation" means maintaining tissues, cells, microorganisms in conditions suitable for their survival and/or growth. Culturing cells may maintain the cell number without any substantial increase of cell number (survival of the cells), but may also mean increasing the cell number by expansion/proliferation of the cells.

Culturing NK cells may include providing the chemical and physical conditions (e.g., temperature, gas) which are required for NK cell maintenance, and growth factors. Often culturing the NK cells includes providing the NK cells with conditions for expansion (proliferation). Examples of chemical conditions which may support NK cell expansion include but are not limited to buffers, serum, nutrients, vitamins, antibiotics, cytokines and other growth factors which are regularly provided in (or may be given manually to) the cell culture medium suited for NK cell expansion. In one embodiment, the NK cell culture medium includes TexMACS Research Medium (Miltenyi Biotec GmbH) supplemented with 5% human serum type AB (Life Technologies) and 500 U/mL of IL-2 (Proleukin S, Novartis). In another embodiment the NK cell culture medium includes Stem Cell Growth Medium SCGM (Cell Genix) supplemented with 5% human serum type AB (Life Technologies) and 500 U/mL of IL-2 (Proleukin S, Novartis). Other media suitable for use to expand NK cells are well known in the art. In another embodiment, the NK cell culture medium includes NK MACS Medium (Miltenyi Biotec, Germany) supplemented with 5% heat inactivated human serum type AB (Access Cell Culture, Vista, CA, USA), and together with cytokines, such as 500 U/mL of human IL-2 (Miltenyi Biotec, Germany), 140 U/mL of human IL-15 (Miltenyi Biotec, Germany). In some embodiments, feeder cells or artificial antigen presenting cells (APCs) such as K562-based feeder cells, lymphoblastoid cell lines, PMBCs, and/or cell-free derivatives such as cell particles or exosomes may be used to expand the composition enriched for NK cells. In some embodiments, the composition enriched for NK cells may be firstly primed with a cytokine from human IL-1 family, such as 2000 U/mL of IL-1beta and/or 50 ng/mL of IL-18 (Miltenyi Biotec, Germany) for 12 to 48 hours before expansion. In some embodiments, the composition enriched for NK cells may be firstly primed with 10 ng/ml of human IL-12, 50 ng/mL of human IL-18, and 50 ng/ml of human IL-15 (Miltenyi Biotec, Germany) for 12 to 18 hours before expansion. In some embodiments, the composition enriched for NK cells may be firstly stimulated with stimulating reagents with stimulating reagents, such as NK cell activation/expansion kit from Miltenyi Biotec, ImmunoCult^{™} NK Cell Expansion Kit from StemCell Technologies, CellXVivo Human NK Cell Expansion Kit from R&D Systems, before expansion. As used herein, the term "expansion" or "proliferation" refers to cell growth and multiplication of cell numbers. Expansion or proliferation, as used herein relate to increased numbers of NK cells occurring during the cultivation process.

As used herein, "essentially free," in terms of a specified component, is used herein to mean that none of the specified component has been purposefully formulated into a composition and/or is present only as a contaminant or in trace amounts. The total amount of the specified component resulting from any unintended contamination of a composition is therefore well below 0.05%, preferably below 0.01%. Most preferred is a composition in which no amount of the specified component can be detected with standard analytical methods.

The term (Pharmaceutical) formulation is the process in which different chemical substances, including the active drug, herein normally the modified NK cells as disclosed herein, are combined to produce a final medicinal product. The word formulation is often used in a way that includes dosage form.

The cancer to be treated as disclosed herein , may be a solid cancer or may be a lymphoma or a hematological malignancy.

Said solid cancer (tumor) may be adrenal cancer, anal cancer, bile duct cancer, bladder cancer, bone cancer, brain/CNS tumors in children or adults, breast cancer, cervical cancer, colon/rectum cancer, endometrial cancer, esophagus cancer, ewing family of tumors, eye cancer, gallbladder cancer, gastrointestinal carcinoid tumors, gastrointestinal stromal tumor (GIST), gestation trophoblastic disease, hodgkin disease, kaposi sarcoma, kidney cancer, laryngeal and hypopharyngeal cancer, leukemia, acute lymphocytic leukemia, acute myeloid leukemia, chronic lymphocytic leukemia, chronic myeloid leukemia, chronic myelomonocytic leukemia, liver cancer, lung cancer, non-small cell lung cancer, small cell lung cancer, lung carcinoid tumor, lymphoma, malignant mesothelioma, multiple myeloma, myelodysplastic syndrome, nasal cavity and paranasal sinum cancer, nasopharyngeal cancer, neuroblastoma, non-hodgkin lymphoma, oral cavity or oropharyngeal cancer, osteosarcoma, ovarian cancer, pancreatic cancer, penile cancer, prostate cancer, rhabdomyosarcoma, , skin cancer, melanoma, merkel cell skin cancer, small intestine cancer, stomach cancer, testicular cancer, thyroid cancer, uterine sarcoma, vaginal cancer, vulvar cancer, or nephroblastoma.

Autoimmune diseases are a condition arising from autoimmunity or disbalance in the immune homeostasis resulting in pathologies that can affect multiple different organ systems. Examples include Behcet's disease, Juvenile idiopathic arthritis, Type 1 diabetes, Rheumatoid arthritis, Wegener Granulomatosis, Systemic lupus erythematosus, Systemic sclerosis, Crohn's disease, Graves' disease, Hashimoto thyroiditis, Goodpasture syndrome, Primary biliary cholangitis, Myasthenia gravis, Dermato polymyositis, Vasculitis, Mixed connective tissue disease, Scleroderma, Multiple sclerosis, Psoriasis, Ulcerative colitis and Uvetis.

Infection (infectious disease) is the invasion of an organism's body tissues by disease-causing agents, their multiplication, and the reaction of host tissues to the infectious agents and the toxins they produce. Infections are caused by infectious agents (pathogens) including: viruses, bacteria, fungi and parasites. Said infection may be an acute or a chronic infection.

Platelets (or thrombocytes) are a blood component whose function (along with the coagulation factors) is to react to bleeding from blood vessel injury by clumping, thereby initiating a blood clot. Resting platelets are small discoid shapes around 1.5-3 µm in size. Platelets have no cell nucleus; they are small fragments of cytoplasm derived from the megakaryocytes of the bone marrow or lung, which then enter the circulation. Platelets are found only in mammals.

The term "spinoculation", or also called "centrifugal inoculation", means a technique used to enhance the efficiency of (retro-)viral transduction. The spinoculation process means centrifuging retroviral vectors such as lentiviral vectors and cells such as NK cells that are mixed together in a suitable solution (e.g. cell medium) at a specific speed and duration, e.g. at 400 x g for 2 hours .

The term "transduction" means the transfer of genetic material from a viral agent such as a retroviral vector such as a lentiviral vector into an eukaryotic cell such as a T cell or an NK cell. The transduction process is the whole process of uptake of the genetic material (the nucleic acid) from a viral agent into a eukaryotic cell (target cell) such as T cell or NK cell and the integration of the genetic material into the genome of said eukaryotic cell, if the viral agent is a retroviral vector such as a lentiviral vector.

The transduction process typically may comprise the following steps:
1. Mix target cells such as NK cells and retroviral vectors such as lentiviral vectors together in a suitable medium such as e.g. cell medium.
2. Apply conditions that facilitate the uptake of retroviral vectors such as lentiviral vectors by target cells such as NK cells. In some embodiments, "spinoculation" (a spinoculation step) at a specific speed (e.g. 400 x g for up to several hours (e.g. 2 hours) will be applied to the mixture of retroviral vectors such as lentiviral vectors and target cells such as NK cells.
3. Complete integration of retroviral vectors such as lentiviral vectors into the target cell genome such as the NK cell genome can take up to 3 days (72 hours) after contact with the target cells (Uchida et al. 2016, Exp Hematol., 44(2):106-15.).

This means the transduction process occurs up to 72 hours after contacting/adding the retroviral vectors such as lentiviral vectors to targets cells such as NK cells. A spinoculation step enhances the efficacy of transduction. Therefore, said transduction process may include 1 or more (e.g. 2, 3, 4 or 5) spinoculation steps within said period of up to 72 hours wherein an uptake and genome integration of the retroviral vector such as lentiviral vector into the target cells such as NK cells can occur.

This means that the process of transduction ends after about 72 hours after contact/addition of the retroviral vector such as lentiviral vector to the target cells such as NK cells under conditions that allow transfer/delivery/uptake of the genetic material of the retroviral vector such as lentiviral vector into the target cells such as NK cells and the integration of said genetic material into the genome of the target cell . The end of the transduction process may be the begin of the cultivation (process).

The step of genetic modification of target cells such as NK cells may end when more than 50%, 60%, 70%, 80%, or 90% of cell medium/solution that allows for the transduction process by a retroviral vector such as a lentiviral vector is exchanged by a cell medium/solution that does not comprise the retroviral vector (e.g. a washing buffer, cell medium) during the coincubation of retroviral vector and target cell, thereby washing out said retroviral vector from the solution (cell medium) that comprises the targets cells such as NK cells.

The term "isolated" is used herein to indicate that the polypeptide, nucleic acid or host cell exist in a physical milieu distinct from that in which it occurs in nature. For example, the isolated polypeptide may be substantially isolated (for example enriched or purified) with respect to the complex cellular milieu in which it naturally occurs, such as in a crude extract.

A transgene may be a gene that has been transferred by genetic engineering techniques into a host cell that normally does not bear this gene. The gene may be a naturally gene that occurs in other cells or may be a recombinant gene. Exemplarily transgenes may be chimeric antigen receptor specific for tumor specific antigen expressed on the surface of a cancer cell. The expressed transgene may also be referred to as a transgene product, a heterologous protein or transgenic polypeptide.

In general, a CAR as used herein may comprise an extracellular domain (extracellular part) comprising the antigen binding domain, a transmembrane domain and a cytoplasmic signaling domain (intracellular signaling domain). The extracellular domain may be linked to the transmembrane domain by a linker or spacer. The extracellular domain may also comprise a signal peptide. In some embodiments of the invention the antigen binding domain of a CAR binds a tag or hapten that is coupled to a polypeptide ("haptenylated" or "tagged" polypeptide), wherein the polypeptide may bind to a disease-associated antigen such as a tumor associated antigen (TAA) that may be expressed on the surface of a cancer cell. Such a CAR may be referred to as "anti-tag" CAR or "adapterCAR" or "universal CAR" as disclosed e.g. in US9233125B2.

The haptens or tags may be coupled directly or indirectly to a polypeptide (the tagged polypeptide), wherein the polypeptide may bind to said disease associated antigen expressed on the (cell) surface of a target. The tag may be e.g. dextran or a hapten such as biotin or fluorescein isothiocyanate (FITC) or phycoerythrin (PE) or thiamin, but the tag may also be a peptide sequence e.g. chemically or recombinantly coupled to the polypeptide part of the tagged polypeptide. The tag may also be streptavidin. The tag portion of the tagged polypeptide is only constrained by being a molecular component that can be recognized and specifically bound by the antigen binding domain specific for the tag of the CAR. For example, when the tag is FITC (fluorescein isothiocyanate), the tag-binding domain may constitute an anti-FITC scFv. Alternatively, when the tag is biotin or PE (phycoerythrin), the tag-binding domain may constitute an anti-biotin scFv or an anti-PE scFv, respectively.

A "signal peptide" refers to a peptide sequence that directs the transport and localization of the protein within a cell, e.g. to a certain cell organelle (such as the endoplasmic reticulum) and/or to the cell surface.

Generally, an "antigen binding domain" in the context of a CAR refers to the region of the CAR that specifically binds to an antigen, e.g. to a tumor associated antigen (TAA) or tumor specific antigen (TSA). The CARs of the invention may comprise one or more antigen binding domains (e.g. a tandem CAR). Generally, the targeting regions on the CAR are extracellular. Generally, the antigen binding domain may comprise an antibody or an antigen binding fragment thereof. The antigen binding domain may comprise, for example, full length heavy chain, Fab fragments, single chain Fv (scFv) fragments, divalent single chain antibodies, diabodies or single domain antibodies (VHHs or nanobodies). The CAR as disclosed herein may comprise a binder as antigen binding domain based on the PDZ3 domain of the tight junction protein ZO-1 as disclosed herein.

Often the antigen binding domain is a scFv or single domain antibody. Normally, in a scFv, the variable regions of an immunoglobulin heavy chain and light chain are fused by a flexible linker to form a scFv. Such a linker may be for example the "(G₄/S)₃-linker".

"Spacer" or "hinge" as used herein refers to the hydrophilic region which is between the antigen binding domain and the transmembrane domain. The CARs of the invention may comprise an extracellular spacer domain but it is also possible to leave out such a spacer. The spacer may include e.g. Fc fragments of antibodies or fragments thereof, hinge regions of antibodies or fragments thereof, CH2 or CH3 regions of antibodies, accessory proteins, artificial spacer sequences or combinations thereof. A prominent example of a spacer is the CD8alpha hinge.

The transmembrane domain of the CAR may be derived from any desired natural or synthetic source for such domain. When the source is natural the domain may be derived from any membrane-bound or transmembrane protein. The transmembrane domain may be derived for example from CD8alpha or CD28. When the key signaling and antigen recognition modules (domains) are on two (or even more) polypeptides then the CAR may have two (or more) transmembrane domains. Separating the key signaling and antigen recognition modules on two different polypeptide chains enables small molecule-dependent, titratable and reversible control over CAR signaling (e.g. WO2014127261A1) due to small molecule-dependent heterodimerizing domains in each polypeptide of the CAR.

The cytoplasmic signaling domain (the intracellular signaling domain or the activating endodomain) of the CAR is responsible for activation of at least one of the normal effector functions of the immune cell in which the CAR is expressed, if the respective CAR is an activating CAR (normally, a CAR as described herein refers to an activating CAR, otherwise it is indicated explicitly as an inhibitory CAR (iCAR)). "Effector function" means a specialized function of a cell, e.g. in a T cell an effector function may be cytolytic activity or helper activity including the secretion of cytokines. The intracellular signaling domain refers to the part of a protein which transduces the effector function signal and directs the cell expressing the CAR to perform a specialized function. The intracellular signaling domain may include any complete, mutated or truncated part of the intracellular signaling domain of a given protein sufficient to transduce a signal which initiates or blocks immune cell effector functions.

Prominent examples of intracellular signaling domains for use in the CARs include the cytoplasmic signaling sequences of the T cell receptor (TCR) complex, co-receptors and co-stimulatory receptors that initiate signal transduction following receptor engagement.

Generally, T cell activation can be mediated by two distinct classes of cytoplasmic signaling sequences, firstly those that initiate antigen-dependent primary activation through the TCR complex (primary cytoplasmic signaling sequences, primary cytoplasmic signaling domain) and secondly those that usually act in an antigen-independent manner to provide a secondary or co-stimulatory signal (secondary cytoplasmic signaling sequences, co-stimulatory signaling domain). Therefore, an intracellular signaling domain of a CAR may comprise one or more primary cytoplasmic signaling domains and/or one or more secondary cytoplasmic signaling domains.

Primary cytoplasmic signaling domains that act in a stimulatory manner may contain ITAMs (immunoreceptor tyrosine-based activation motifs).

Examples of ITAM containing primary cytoplasmic signaling domains often used in CARs are those derived from TCRζ (CD3ζ), FcRgamma, FcRbeta, CD3gamma, CD3delta, CD3epsilon, CD5, CD22, CD79a, CD79b, and CD66d. Most prominent is sequence derived from CD3ζ.

The cytoplasmic domain of the CAR may be designed to comprise the CD3ζ signaling domain by itself or combined with any other desired cytoplasmic domain(s). The cytoplasmic domain of the CAR can comprise a CD3ζ chain portion and a co-stimulatory signaling region (domain). The co-stimulatory signaling region refers to a part of the CAR comprising the intracellular domain of a co-stimulatory molecule. A co-stimulatory molecule is a cell surface molecule other than an antigen receptor or their ligands that is required for an efficient response of lymphocytes to an antigen. Examples for a co-stimulatory molecule are CD27, CD28, 4-1BB (CD137), OX40, CD30, CD40, ICOS, lymphocyte function-associated antigen- 1 (LFA-1), CD2, CD7, LIGHT, NKG2C, B7-H3.

The cytoplasmic signaling sequences within the cytoplasmic signaling part of the CAR may be linked to each other with or without a linker in a random or specified order. A short oligo- or polypeptide linker, which is preferably between 2 and 10 amino acids in length, may form the linkage. A prominent linker is the glycine-serine doublet.

As an example, the cytoplasmic domain may comprise the signaling domain of CD3ζ and the signaling domain of CD28. In another example, the cytoplasmic domain may comprise the signaling domain of CD3ζ and the signaling domain of CD137. In a further example, the cytoplasmic domain may comprise the signaling domain of CD3ζ, the signaling domain of CD28, and the signaling domain of CD137.

As aforementioned either the extracellular part or the transmembrane domain or the cytoplasmic domain of a CAR may also comprise a heterodimerizing domain for the aim of separating key signaling and antigen recognition modules of the CAR onto two (or more) separate polypeptide chains.

The CAR may be further modified to include on the level of the nucleic acid encoding the CAR one or more operative elements to eliminate CAR expressing immune cells by virtue of a suicide switch. The suicide switch can include, for example, an apoptosis inducing signaling cascade or a drug that induces cell death. In one embodiment, the nucleic acid expressing and encoding the CAR can be further modified to express an enzyme such thymidine kinase (TK) or cytosine deaminase (CD). The CAR may also be part of a gene expression system that allows controlled expression of the CAR in the immune cell. Such a gene expression system may be an inducible gene expression system and wherein when an induction agent is administered to a cell being transduced with said inducible gene expression system, the gene expression system is induced and said CAR is expressed on the surface of said transduced cell.

In some embodiments, the endodomain may contain a primary cytoplasmic signaling domain or a co-stimulatory region, but not both.

In some embodiments, the CAR may be a "SUPRA" (split, universal, and programmable) CAR, where a "zipCAR" domain may link an intracellular co-stimulatory domain and an extracellular leucine zipper (WO2017/091546). This zipper may be targeted with a complementary zipper fused e.g. to an scFv region to render the SUPRA CAR T cell tumor specific. This approach would be particularly useful for generating universal CAR T cells for various tumors; adapter molecules could be designed for tumor specificity and would provide options for altering specificity post-adoptive transfer, key for situations of selection pressure and antigen escape.

The CARs as described herein may be designed to comprise any portion or part of the above-mentioned domains as described herein in any order and/or combination resulting in a functional CAR, i.e. a CAR that mediated an immune effector response of the immune effector cell that expresses the CAR as disclosed herein.

The term "tagged polypeptide" as used herein refers to a polypeptide that has bound thereto directly or indirectly at least one additional component, i.e. the tag. The tagged polypeptide as used herein is able to bind an antigen expressed on a target cell. The polypeptide may be an antibody or antigen binding fragment thereof that binds to an antigen expressed on the surface of a target cell such as a tumor associated antigen on a cancer cell. The polypeptide of the tagged polypeptide alternatively may be a cytokine or a growth factor or another soluble polypeptide that is capable of binding to an antigen of a target cell.

The terms "adapter" or "adapter molecule" or "tagged polypeptide" as used herein may be used interchangeably.

The tag may be e.g. a hapten or dextran and the hapten or dextran may be bound by the antigen binding domain of the polypeptide, e.g. a CAR, comprising an antigen binding domain specific for the tag.

Haptens such as e.g. FITC, biotin, or dextran are small molecules that elicit an immune response only when attached to a large carrier such as a protein; the carrier may be one that also does not elicit an immune response by itself. Once the body has generated antibodies to a hapten-carrier adduct, the small-molecule hapten may also be able to bind to the antibody, but it will usually not initiate an immune response; usually only the hapten-carrier adduct can do this.

However, the tag may also be a peptide sequence a (peptide) e.g. chemically or recombinantly coupled to the polypeptide part of the tagged polypeptide. The peptide may be e.g. c-Myc-tag, Strep-Tag, Flag-Tag, and Polyhistidine-tag. The tag may also be streptavidin. The tag portion of the tagged polypeptide is only constrained by being a molecular component that can be recognized and specifically bound by the antigen binding domain specific for the tag of the CAR. For example, when the tag is FITC (Fluorescein isothiocyanate), the tag-binding domain may constitute an anti-FITC scFv. Alternatively, when the tag is biotin or PE (phycoerythrin), the tag-binding domain may constitute an anti-biotin scFv or an anti-PE scFv.

The T cell receptor (TCR) is a protein complex found on the surface of T cells, or T lymphocytes, that is responsible for recognizing fragments of antigen as peptides bound to major histocompatibility complex (MHC) molecules. However, TCRs can also be non-MHC-restricted, such as most γδ TCRs.

The TCR is composed of two different protein chains (that is, it is a heterodimer). In humans, in 95% of T cells the TCR consists of an alpha (α) chain and a beta (β) chain (encoded by TRA and TRB, respectively), whereas in 5% of T cells the TCR consists of gamma and delta (γ/δ) chains (encoded by TRG and TRD, respectively). This ratio changes during ontogeny and in diseased states (such as leukemia). Each locus can produce a variety of polypeptides with constant and variable regions. A transgene encoding a TCR may also be referred to as a transgenic TCR (tTCR).

### Examples

The following examples are intended for a more detailed explanation of the invention but without restricting the invention to these examples.

### Example 1: The presence of dasatinib during the transduction process suppresses the expression of NKG2D ligands that are upregulated on NK cells upon viral transduction.

Natural Killer Group 2, member D (NKG2D) receptor is a single-pass type II membrane protein, which is encoded by killer cell lectin like receptor K1 (KLRK1) gene. NKG2D receptor is an activating receptor and its expression has been found in NK cells and subsets of T cells. Human NKG2D receptor has an extracellular C-type lectin-like domain and can recognize multiple membrane-anchored ligands, including MICA, MICB and six UL16 binding proteins (ULBP1-6). In general, NKG2D ligands (NKG2DLs) are not expressed on the surface of healthy human adult cells, but can be upregulated in response to cellular stress, such as viral infection or transformation of cells. Human NKG2D represents as a homodimer that associates with DAP10 dimers as signaling proteins. Engagement of NKG2D by its ligands is sufficient to trigger the activation and degranulation of NK cells, resulting in the lysis of target cells expressing NKG2DLs.

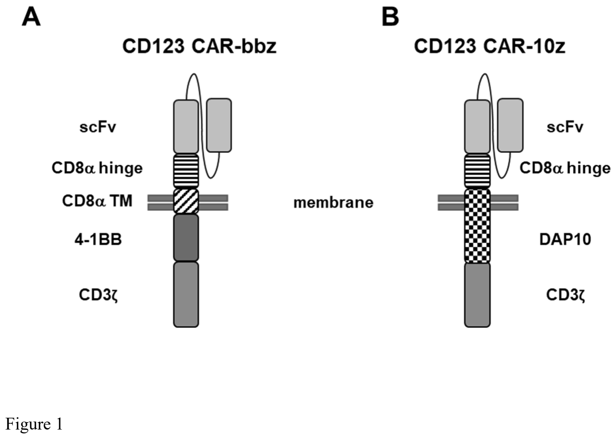

Two CD123-specific CARs (CD123 CAR), CD123 CAR-bbz and CD123 CAR-10z, were designed and generated to be used as genes of interest in the present invention. The CD123 CAR-bbz sequence consists of a human granulocyte macrophage colony stimulating factor receptor alpha subunit (GM-CSFRα) signal peptide, a CD123-specific single chain Fv (scFv) antibody fragment, a CD8α hinge and transmembrane (TM) domain, followed by 4-1BB and CD3ζ intracellular (IC) domains (Figure 1A). The CD123 CAR-10z sequence consists of a human GM-CSFRα signal peptide, a CD123-specific scFv antibody fragment, a CD8α hinge, followed DAP10 TM and IC domains and CD3ζ IC domain (Figure 1B). Baboon envelope pseudotyped lentiviral vectors (BaEV-LVs) containing supernatants were produced by transient transfection of HEK 293T cells. LV-containing supernatants were stored at -80°C before use.

Peripheral blood mononuclear cells (PBMC) were isolated from healthy donors by Pancoll (Pan biotech, Aidenbach, Germany) density gradient centrifugation. Primary human NK cells were enriched from PBMCs with CD3-positive cell depletion followed by CD56-positive cell enrichment using antibody coated microbeads (Miltenyi Biotec). The isolated NK cells were cultured in NK cell culture medium containing NK MACS medium (Miltenyi Biotec), supplemented with 5% heat inactivated AB serum (Access Cell Culture, Vista, CA, USA), 500 IU/mL human IL-2 and/or 140 IU/mL human IL-15 (Miltenyi Biotec). On day 1, NK cells were transduced with BaEV-LV encoding the CD123 CARs in the presence of vectofusin-1, including a step of 2 h spinoculation at 400 x g, 32°C. Culture medium was exchanged every 2-3 days. Untransduced (UTD) NK cells were subjected to centrifugation in the same way as transduced NK cells, but in the absence of lentiviral vectors. The surface expression of NKG2D ligands (NKG2DLs) on NK cells were determined over time with flow cytometric analysis. The NKG2DL expression was detected by using recombinant human NKG2D-His-tag protein (Acro biosystems, DE, USA) followed by PE-conjugated anti-His-tag antibody (Miltenyi Biotec).

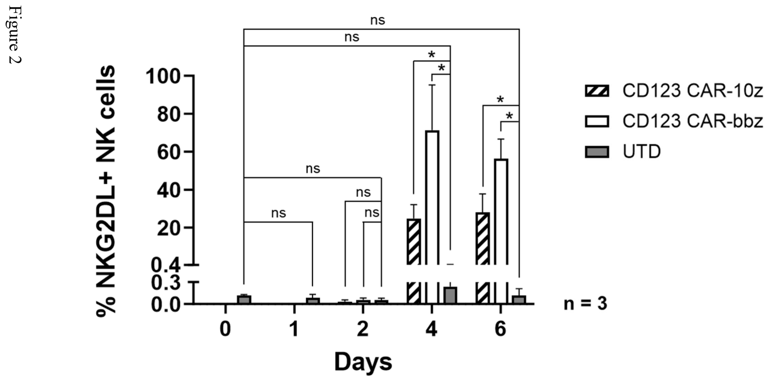

Freshly isolated and overnight cultured NK cells showed very low levels of the NKG2DL expression prior to transduction (Day 0 and Day 1 in Figure 2, respectively). No significant upregulation of the NKG2DL expression was observed on day 2. However, the expressions of NKG2DLs were significantly increased 3 days after the exposure to viral vectors for both CD123 CAR-bbz and CD123 CAR-10z NK cells compared with UTD NK cells. The elevated NKG2DL expressions on CD123 CAR NK cells were maintained on day 6. In contrast, no significant increase of NKG2DLs in UTD NK cells was detected during the experiment, compared with the freshly isolated NK cells, demonstrating that spinoculation did not significantly increase the expression of NKG2DLs on NK cells.

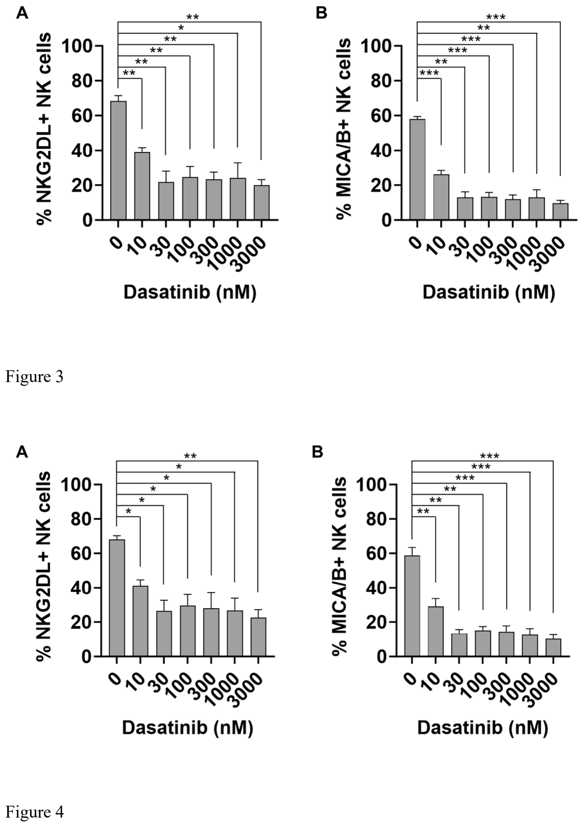

In order to determine the effect of dasatinib on the expression of NKG2DLs on gene-modified NK cells, NK cells were transduced on day 1 with BaEV-LV encoding the CD123 CARs in the presence of vectofusin-1, including a step of 2 h spinoculation at 400 x g, 32°C. Dasatinib was added to the CD123 CAR NK cells during the transduction process. The dasatinib concentration ranged from 10 to 3000 nM. CD123 CAR NK cells treated without any dasatinib served as controls. The expressions of NKG2DLs and major histocompatibility complex (MHC) class I chain-related protein A and B (MICA/B) were determined on day 4. In brief, the NKG2DL expression was detected by using biotinylated human recombinant NKG2D-Fc protein (Acro biosystems, DE, USA) and PE-conjugated streptavidin (Miltenyi Biotec) and the MICA/B expression was detected by using PE-Vio 770-conjugated anti-human MICA/MICB antibody (Miltenyi Biotec). The expressions of NKG2DLs and MICA/B were significantly reduced for both CD123 CAR-bbz (Figure 3) and CD123 CAR-10z NK cells (Figure 4) that were treated with dasatinib at various concentrations during the transduction process compared to those treated with no dasatinib.

The data summarized in this example demonstrate that NKG2D ligands are upregulated on the NK cell surface during gene modification. However, the presence of dasatinib during the transduction process can effectively reduce the surface expression of NKG2DLs on gene-modified NK cells.

### Example 2: The presence of dasatinib during the transduction process improves the viability of gene-modified NK cells.

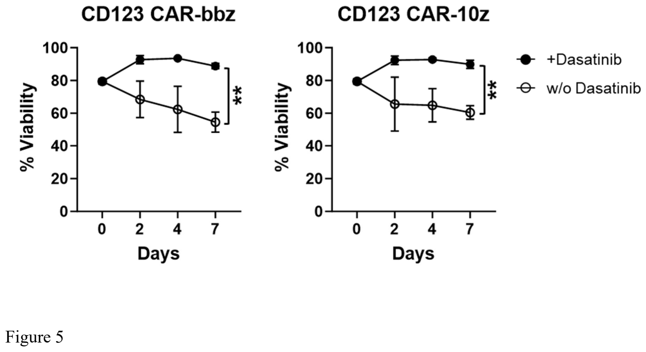

On day 0, 100 nM of dasatinib was added to the culture of freshly-isolated NK cells. CD123 CAR-bbz and CD123 CAR-10z NK cells were produced as described in Example 1. CAR NK cells with no dasatinib treatment served as controls. NK cell viability was monitored for 7 days. The viability of CAR NK cells was analyzed by staining with 7-AAD (Miltenyi Biotec) and determined with a MACSQuant Analyzer 10 flow cytometer. Both CD123 CAR-bbz and CD123 CAR-10z cells generated with the presence of dasatinib during the transduction process showed significantly higher viability, compared with those treated without dasatinib (Figure 5). The data summarized in this example demonstrate that the presence of dasatinib during the transduction process can improve the viability of NK cells subjected to viral transduction.

### Example 3: CAR NK cells generated with the presence of dasatinib during the transduction process display potent in vitro antitumor activity.

To assess the effect of the presence of dasatinib during the transduction process on the antitumor activity of CAR NK cells, CD123 CAR-bbz cells were produced using NK cells derived from three different healthy donors as described in Example 1. NK cells were treated with or without 100 nM of dasatinib during the transduction process. UTD NK cells were subjected to centrifugation in the same way as transduced NK cells, but in the absence of lentiviral vectors. On day 13, CAR NK cells were coincubated with 3 x 10⁴ of CD123+/GFP+ THP-1 tumor cells at an E:T ratio of 1:3, which was calculated based on the number of transduced NK cells. CAR NK cells were then repeatedly challenged for 7 days with 3 x 10⁴ of fresh THP-1 tumor cells every 48 hours. The cytotoxicity assay was performed in RPMI1640 medium supplemented with 10% fetal bovine serum (FBS) and 2 mM L-Glutamine in the absence of cytokines in favor of tumor cell growth. Tumor cells incubated without NK cells and with UTD NK cells were prepared to serve as controls. The cytolytic activity over time was evaluated by real-time monitoring using the IncuCyte S3 system (Sartorius, Germany).

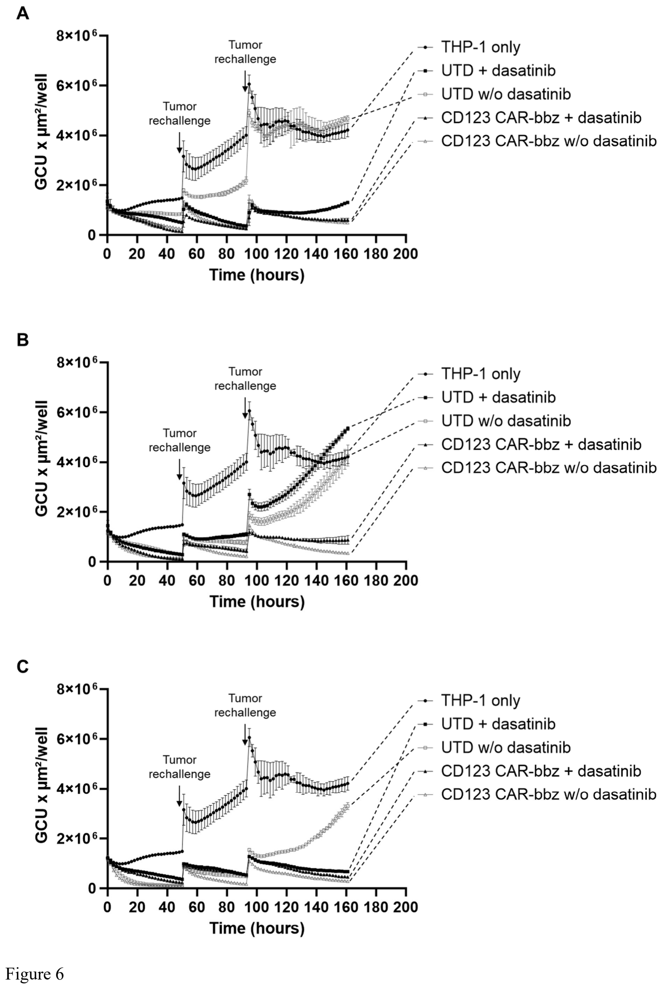

The tumor cells rapidly grew in the samples coincubated without NK cells in all three experiments. Both UTD NK cells and CAR NK cells displayed antitumor activity. Intriguingly, dasatinib treatment during the transduction process did not result in a reduction in the cytotoxicity of UTD NK cells against tumor cells. In contrast, it led to a notable enhancement in the antitumor activity of UTD NK cells in two out of three experiments. Moreover, dasatinib treatment did not impair CAR NK cell cytotoxicity, resulting in comparable strong inhibition of tumor growth for both CD123 CAR-bbz NK cells generated with or without the presence of dasatinib during the transduction process until the end of the experiment, despite two rounds of tumor rechallenge (Figure 6).

The data summarized in this example demonstrate that the presence of dasatinib during the transduction process does not reduce NK cell cytotoxicity, thereby substantiating the absence of a dasatinib-induced deactivation effect on the final NK cell products.

### Example 4: The presence of dasatinib during the transduction process results in enhanced in vivo antitumor activity of gene-modified NK cells.

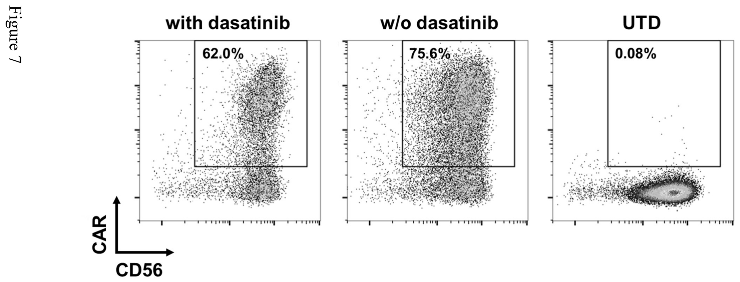

The antitumor activity of CAR NK cells generated with the presence of dasatinib during the transduction process was further assessed in a THP-1 xenograft NOD-SCID IL2R γ^{null} (NSG) mouse model. Primary human NK cells were isolated and cultured in NK cell culture medium as described in Example 1. On day 2, NK cells were transduced with BaEV-LV encoding the CD123 CAR-bbz construct in the presence of vectofusin-1 to generate CD123 CAR-bbz NK cells. NK cells were treated 100nM of dasatinib during the transduction process. CD123 CAR-bbz NK cells treated without dasatinib served as controls. CAR NK cells were cultured until day 14. CAR expression was detected by using human CD123 CAR detection reagent and VioBright 515-conjugated anti-biotin antibody (Miltenyi Biotec). A slight reduction in CAR expression was observed in NK cells treated with dasatinib during the transduction process, compared to those without dasatinib treatment (Figure 7).

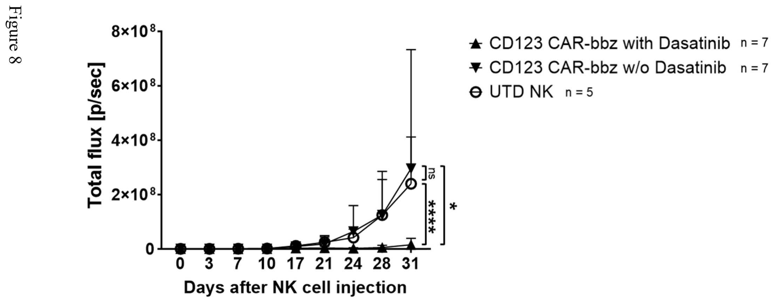

NSG mice were intravenously injected with 1 x 10⁶ THP-1 cells that were engineered to express green fluorescent protein (GFP) and luciferase (Luc). Seven days after tumor inoculation, the mice received a single administration of 5 x 10⁶ CD123 CAR-bbz NK cells generated with or without the presence of dasatinib during the transduction process. Instead, the mice in the control group received UTD NK cells. While tumors grew gradually in the mice treated with UTD NK cells or CD123 CAR-bbz NK cells without dasatinib treatment during the transduction process, CD123 CAR-bbz NK cells with dasatinib treatment during the transduction process significantly suppressed tumor growth in mice (Figure 8). No weight loss, distress, or other abnormalities was observed for the mice during the experiment.

### Example 5: The effect of the presence of dasatinib during the transduction process on the proliferation of gene-modified NK cells is dose-dependent.

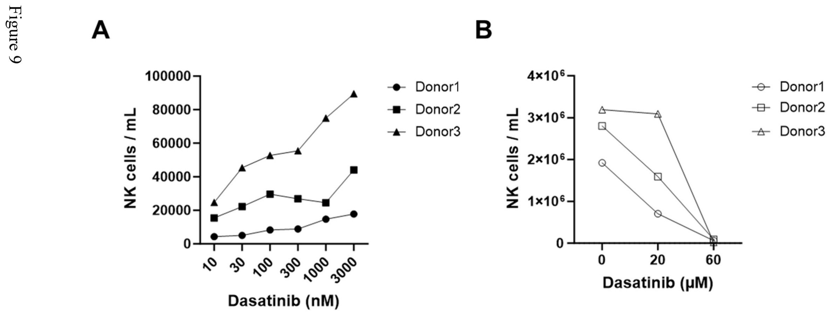

To determine the effect of the presence of dasatinib during the transduction process on the proliferation of gene-modified NK cells, CAR NK cells were generated from freshly isolated primary human NK cells as described in Example 1. NK cells were treated with different concentrations of dasatinib as indicated in Figure 9A during the transduction process. CAR NK cells were cultured until day 8 and viable NK cell numbers were counted by using a MACSQuant Analyzer 10 flow cytometer. Higher NK cell numbers could be obtained from the samples treated with higher concentrations of dasatinib from all three donors (Figure 9A), indicating the beneficial effect of dasatinib treatment during transduction process on the proliferation of NK cells. However, further increasing the concentration of dasatinib to 20 µM had a negative effect on NK cell proliferation in another experiment and treatment of CAR NK cells with 60 µM dasatinib resulted in no viable NK cells at the end of the experiment (Figure 9B).

### Example 6: Dasatinib does not appear to benefit NK cell electroporation process.

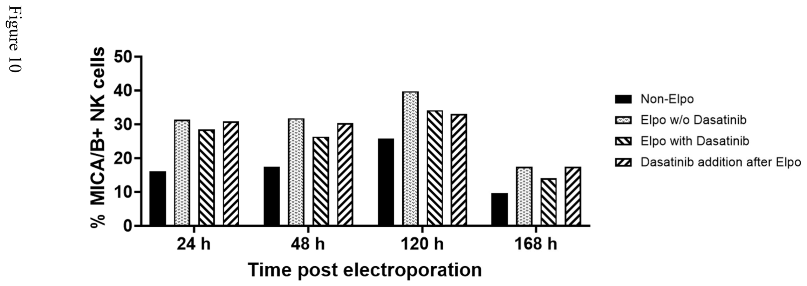

Electroporation (Elpo) is a technique in which an electrical field is applied to cells in order to increase the permeability of the cell membrane, allowing chemicals, drugs, protein and/or DNA to be introduced into the cell. To determine the effect of dasatinib on electroporation, NK cells were seeded as 1×10⁶ cells in 1 mL of NK MACS medium as previously described (Albinger et al. 2024, Bone Marrow Transplant., 59(4):489-495.) with or without 100 nM dasatinib. Sixteen hours after seeding, NK cells were electroporated by a CliniMACS^{®} Electroporator (Miltenyi Biotec B.V. & Co. KG, Germany) with 2 µg of EGFR mRNAs for each sample. After electroporation, all the NK cell samples were maintained in the same medium as before, resulting in the samples of Elpo with dasatinib and Elpo w/o dasatinib NK cells shown in Figure 10. Of note, 100 nM dasatinib was added to an electroporated NK cell sample that had not been exposed to dasatinib prior to electroporation, resulting in the sample of dasatinib addition after Elpo shown in Figure 10. Non-electroporated NK cells (Non-Elpo shown in Figure 10) cultured without dasatinib served as a control. MICA/B expression and viable NK cell counts were determined as described in Example 1.

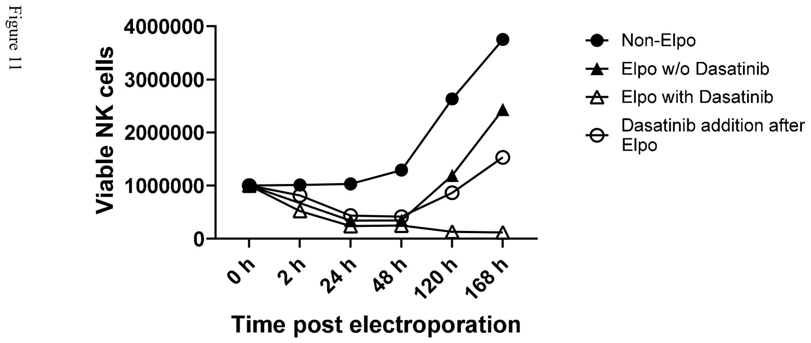

Higher expression of MICA/B was shown on the electroporated NK cells compared to the non-electroporated control, indicating the cellular stress induced by the electroporation process (Figure 10). In contrast to its effect on transduction, however, dasatinib did not markedly reduce the expression of MICA/B on electroporated NK cells. While the addition of dasatinib after the electroporation resulted in a modest decrease in NK cell counts compared to the sample electroporated without dasatinib, the electroporation with dasatinib strongly inhibited the proliferation of NK cells (Figure 11).

### Example 7: Identifying the optimal timing for incorporating dasatinib in CAR NK cell manufacturing.

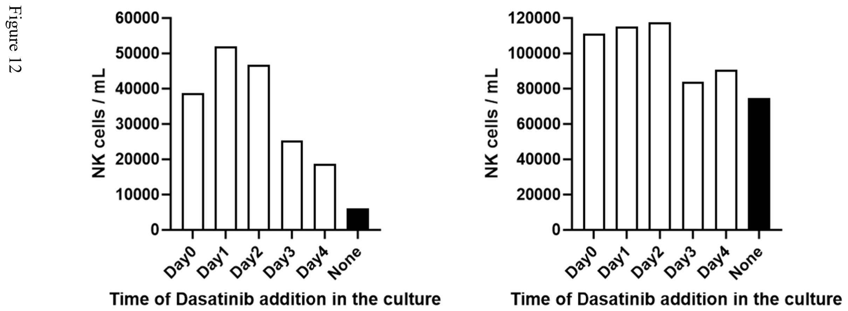

To determine the optimal timing for adding dasatinib in CAR NK cell manufacturing, CD123 CAR-bbz NK cells were generated from freshly isolated primary human NK cells as described in Example 1. NK cells were treated with 100 nm of dasatinib at different time points. CAR NK cells were cultured until day 8 and viable NK cell numbers were counted by using a MACSQuant Analyzer 10 flow cytometer. Earlier dasatinib treatment between days 0-2 resulted in superior expansion of CD123 CAR-bbz NK cells, compared to untreated controls or those receiving dasatinib at latter time points (Figure 12). It demonstrates the beneficial effect on NK cell proliferation when dasatinib is added between up to 24 hours before and no later than 24 hours after NK cells contact LV.

## Claims

1. An in-vitro method for the generation of a composition comprising genetically modified NK cells, the method comprising the steps
a) enrichment of NK cells from a sample comprising NK cells and other cells,
b) genetic modification of said enriched NK cells by transduction with a retroviral vector
c) cultivation of said genetically modified NK cells,
wherein said genetic modification step b) is performed in the presence of a tyrosine kinase (TK) inhibitor, wherein the step of genetic modification of said enriched NK cells comprise the addition of said TK inhibitor to said enriched NK cells not later than 24 hours after the contact of the retroviral vector with the enriched NK cells, and wherein said TK inhibitor is added up to 24 hours before said genetic modification step to said enriched NK cells.

2. The method of claim 1, wherein said TK inhibitor is also present during the cultivation step c).

3. The method of claim 1, wherein said TK inhibitor is not present during the cultivation step c).

4. The method of claims 1 to 3, wherein said method comprises step d):
formulating the genetically-modified and cultured NK cells into a final cell composition, wherein said final cell composition formulation is free of said TK inhibitor.

5. The method of claims 1 to 4, wherein said retroviral vector is a lentiviral vector.

6. The method of claims 1 to 5, wherein said TK inhibitor is dasatinib.

7. The method of claims 1 to 6, wherein the concentration of said TK inhibitor in the liquid that comprises the NK cells in step b) is between 10 nM and 20 µM.
